# FASCICULE DE BREVET EUROPEEN

(11) **EP 2 941 627 B1**
(45) Date de publication et mention de la délivrance du brevet: **05.08.2020**
(21) Numéro de dépôt: 13827026.9
(22) Date de dépôt: 30.12.2013
(51) Int. Cl.: G01G 19/50

(54) **DISPOSITIF DE PESAGE MULTI-FONCTION**
MULTIFUNKTIONSWIEGEVORRICHTUNG
MULTIPURPOSE WEIGHING DEVICE

(30) Priorité: 02.01.2013 FR 1350013
(43) Date de publication de la demande: 11.11.2015
(73) Titulaire: Withings, 92130 Issy-les-Moulineaux (FR)
(72) Inventeur: CARREEL, Eric, F-92190 Meudon (FR); BRAC DE LA PERRIERE, Brice, F-75007 Paris (FR); BUARD, Nadine, F-92190 Meudon (FR); BARROCHIN, Pierre, F-92210 Saint Cloud (FR); YANG, Rui-Yi, F-89000 Auxerre (FR); FAUSSARD, Guillaume, F-78770 Villiers-le-Mahieu (FR); AITMBAREK, Said, F-92160 Antony (FR)
(74) Mandataire: Plasseraud IP
(86) Numéro de dépôt international: PCT/FR2013/053280
(87) Numéro de publication internationale: WO 2014/106716

(56) Documents cités:
- EP-A1- 1 166 716
- EP-A1- 1 314 396
- EP-A1- 1 745 740
- WO-A1-98/13674
- ES-A1- 2 296 474
- ES-A1- 2 328 205

## Description

La présente invention est relative aux dispositifs et procédés de pesage.

Plus particulièrement, l'invention concerne le domaine des pèse-personnes électroniques. Ces appareils sont usuellement équipés de quatre pieds, chacun de ces pieds étant équipé d'une jauge de contrainte. Il a été proposé d'utiliser lesdites jauges de contraintes pour évaluer le rythme cardiaque (la fréquence cardiaque) d'un utilisateur placé sur le pèse-personne, comme par exemple dans le document ES2328205. Cependant, il s'avère que ce procédé peut fonctionner sur une partie de la population mais ne fonctionne pas sur une portion significative des individus de la population.

Par ailleurs, certains pèse-personnes électroniques possèdent une fonction de mesure d'impédance, destinée par exemple à indiquer un indice physiologique de l'utilisateur, comme par exemple le taux de masse graisseuse. Cela nécessite néanmoins que l'utilisateur soit pieds nus sur la balance. Il a été proposé d'utiliser la mesure d'impédance pour évaluer le rythme cardiaque de l'utilisateur comme par exemple dans le document ES2296474. Cependant, il s'avère également que ce procédé peut fonctionner sur une partie de la population mais ne fonctionne pas sur une portion significative des individus de la population, et de plus il faut que l'utilisateur soit pieds nus.

Il est donc apparu un besoin de proposer une solution améliorée pour mesurer le rythme cardiaque d'un individu placé sur un pèse-personne électronique.

A cet effet, l'invention propose notamment un pèse-personne électronique selon la revendication 1.

Grâce à ces dispositions, on obtient une mesure fiabilisée de la fréquence cardiaque d'un individu, qui permet de mesurer la fréquence cardiaque pour la plupart des individus.

Dans des modes de réalisation du procédé selon l'invention, on peut éventuellement avoir recours en outre à l'une et/ou à l'autre des dispositions suivantes :
- le dispositif peut comprendre quatre portions conductrices, et l'unité électronique peut être configurée pour injecter un courant pulsé ou modulé entre deux portions conductrices et mesurer la différence de potentiel entre les deux autres portions conductrices, pour déterminer les variations de différence de potentiel, et déterminer ainsi le deuxième signal cardiaque ; de sorte que l'on évite que ce deuxième signal cardiaque soir parasités par les influx électriques issus des muscles, et la mesure d'impédance peut être fiabilisée sans que le courant moyen injecté ne soit trop important.
- l'unité électronique est configurée pour évaluer un indice de qualité du premier signal et un indice de qualité du deuxième signal, et déterminer la fréquence cardiaque à partir du signal ayant le meilleur indice de qualité ; moyennant quoi on peut toujours choisir les plus pertinents des premier et deuxième signaux afin de déterminer la fréquence cardiaque.
- l'unité électronique est configurée pour réaliser un calcul d'inter-corrélation sur les premier et deuxième signaux ; moyennant quoi on peut combiner avantageusement les premier et deuxième signaux.
- l'unité électronique peut être configurée pour mesurer un déphasage temporel entre les premier et deuxième signaux, et décaler le deuxième signal par rapport au premier signal dudit déphasage temporel ; de cette manière, le calcul d'inter-corrélation élimine les bruits et maximise les portions de signal utiles.
- l'unité électronique est configurée pour évaluer un indice de qualité du calcul d'inter-corrélation, et déterminer la fréquence cardiaque à partir des premier et deuxième signaux et du calcul d'inter-corrélation en choisissant celui des trois ayant le meilleur indice de qualité ; moyennant quoi on maximise le taux de couverture de la mesure pour la plupart des individus.
- le dispositif peut comprendre quatre pieds et quatre jauges de contrainte correspondantes, combinées dans deux ponts de wheatstone, pour permettre à l'unité électronique de mesurer le poids d'un utilisateur et ses variations ; moyennant quoi, le schéma de pesée est optimisé et la mesure de poids est fiabilisée.

L'invention vise aussi un procédé mis en oeuvre dans un pèse-personne électronique comprenant au moins une jauge de contrainte, au moins deux portions conductrices agencées sur une surface supérieure, et une unité électronique de commande, la jauge de contrainte et les portions conductrices étant raccordées à l'unité électronique, pour permettre à l'unité électronique de mesurer le poids d'un utilisateur placé sur le dispositif de pesage, et une impédance aux bornes des pieds de l'utilisateur,

### le procédé comprenant les étapes :

a- mesurer un premier signal représentatif des variations de poids périodiques engendrées par les battements cardiaques de l'utilisateur,
b- mesurer un deuxième signal représentatif des variations d'impédance périodiques engendrées par les battements cardiaques de l'utilisateur,
c0- procéder à une analyse individuelle qualitative des premier et deuxième signaux et une analyse comparée des premier et deuxième signaux,
c- déterminer, à partir des premier et deuxième signaux, la fréquence cardiaque de l'utilisateur,
   de sorte que la mesure et l'affichage de la fréquence cardiaque est fiabilisé par l'analyse combinée des deux signaux.
   - le dispositif peut comprendre quatre portions conductrices, le procédé comprenant, au cours de l'étape b-, l'injection d'un courant pulsé entre deux des portions conductrices et la mesure de la différence de potentiel entre les deux autres portions conductrices, pour déterminer les variations de différence de potentiel, et déterminer ainsi le deuxième signal ; de sorte que la mesure d'impédance peut être fiabilisée sans que le courant moyen injecté ne soit trop important.
   - le procédé peut comprendre en outre, dans l'étape c-, la réalisation d'au moins un calcul d'inter-corrélation sur les premier et deuxième signaux ; moyennant quoi on peut combiner avantageusement les premier et deuxième signaux.
   - le procédé peut comprendre en outre dans l'étape c-la mesure d'un déphasage temporel entre les premier et deuxième signaux, afin de décaler le deuxième signal par rapport au premier signal dudit déphasage temporel, de manière à ce que le calcul d'inter-corrélation élimine les bruits et maximise les portions de signal utiles.

D'autres caractéristiques et avantages de l'invention apparaîtront au cours de la description suivante d'une de ses formes de réalisation, donnée à titre d'exemple non limitatif, en regard des dessins joints.

Sur les dessins :
- la figure 1 est une vue générale en perspective du dispositif de pesage selon l'invention,
- la figure 2 est une vue de dessus schématique du dispositif de la figure 1,
- la figure 3 montre un schéma fonctionnel du dispositif de pesage de la figure 1, et
- la figure 4 montre un schéma électrique de principe du dispositif de pesage de la figure 1,
- la figure 5 montre un chronogramme relatif au procédé de mesure des variations d'impédance,
- les figures 6 et 7 montrent des chronogrammes des premier et deuxième signaux,
- la figure 8 montre un schéma de principe d'un circuit de filtrage et d'amplification,
- la figure 9 montre un schéma système intégrant le dispositif de pesage de la figure 1.

Sur les différentes figures, les mêmes références désignent des éléments identiques ou similaires.

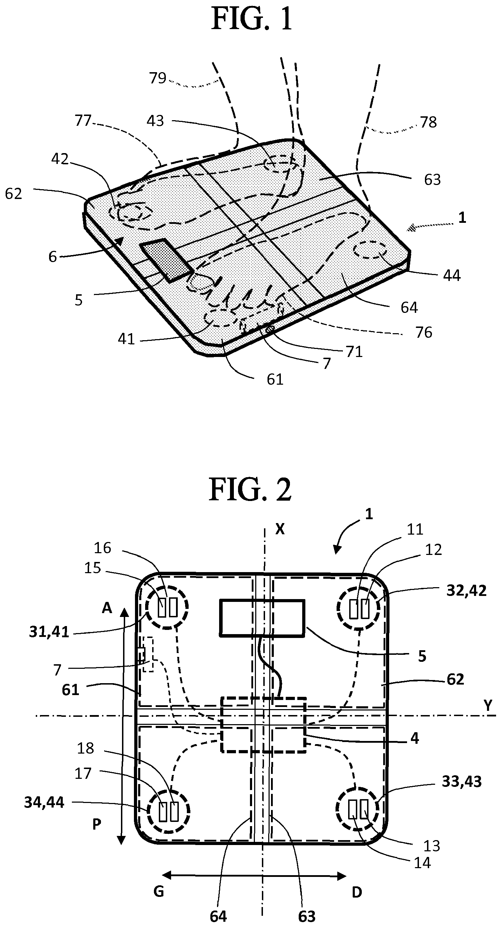

La figure 1 représente un exemple de pèse-personne électronique 1 sur lequel peut se placer un utilisateur pour mesurer notamment son poids. Dans l'exemple illustré, on voit les jambes **78, 79** de l'utilisateur ainsi que ses pieds **76, 77,** qui sont nus comme illustré.

Le pèse-personne électronique **1** comprend un corps principal de forme générale rectangulaire ou carrée et quatre pieds **41-44** disposés respectivement au voisinage des quatre coins du corps, chaque pied comprenant des moyens de mesure.

Plus précisément, selon l'exemple illustré ici, le pied antérieur gauche **41** comprend une jauge de contrainte antérieure gauche **31,** le pied antérieur droit **42** comprend une jauge de contrainte antérieure droite **32,** le pied postérieur droit **43** comprend une jauge de contrainte postérieure droite **33** et le pied postérieur gauche **44** comprend une jauge de contrainte postérieure gauche **34.**

Il est bien entendu que le pèse-personne électronique pourrait aussi comporter un nombre différent de jauges de contrainte, de plus sans relation directe avec le nombre de pieds. En particulier, il pourrait n'y avoir qu'une seule jauge de contrainte centrale, ou bien deux jauges de contrainte, ou bien encore trois jauges de contrainte. Il n'est pas non plus exclu d'avoir plus de quatre jauges de contrainte.

Sur la figure 2, la direction antéro-postérieure est référencée par l'axe **X,** la lettre **A** désignant l'avant et la lettre **P** désignant l'arrière, alors que la direction droite-gauche est référencée par l'axe **Y,** s'étendant entre le côté gauche dénoté **G** et le côté droit dénoté **D.**

Le pèse-personne électronique **1** comprend en outre une unité de commande électronique **4** (aussi nommée 'unité électronique') et un afficheur **5** dont il sera question plus en détail plus loin.

S'agissant des jauges de contrainte **31-34** déjà mentionnées (aussi appelées jauges extensiométriques), elles comprennent chacune un premier élément dont la résistance augmente sous l'effet d'une compression verticale appliquée aux pieds considérés et un deuxième élément dont la résistance diminue sous l'effet de ladite compression verticale.

Dans l'exemple illustré ici, la jauge antérieure droite **32** comprend un tel premier élément **11** appelé première résistance de jauge avant droite 11, et un tel deuxième élément appelé deuxième résistance de jauge avant droite **12.**

De façon analogue, la jauge postérieure droite **33** comprend un tel premier élément appelé première résistance de jauge arrière droite **13,** et un tel deuxième élément appelé deuxième résistance de jauge arrière droite **14.**

De même pour le côté gauche, la jauge antérieure gauche **31** comprend un tel premier élément **15** appelé première résistance de jauge avant gauche 15, et un tel deuxième élément appelé deuxième résistance de jauge avant gauche **16.**

Enfin, la jauge postérieure gauche **34** comprend un tel premier élément appelé première résistance de jauge arrière gauche **17,** et un tel deuxième élément appelé deuxième résistance de jauge arrière gauche **18.**

Chacune des résistances **11-18** présente respectivement une valeur de résistance notée **R1-R8.** Dans l'exemple illustré ici, les résistances impaires augmentent avec l'effort appliqué sur les pieds, alors qu'à l'inverse les résistances paires diminuent avec l'effort appliqué.

La combinaison des huit résistances **11-18** peut prendre plusieurs formes ; une seule sera présentée en détails ci-après, mais d'autres combinaisons sont bien sûr possibles.

De plus, le pèse-personne électronique **1** présente une surface supérieure **6** comprenant quatre portions conductrices **61-64** agencées sur ladite surface supérieure, isolées électriquement l'une de l'autre.

Ces portions conductrices servent d'électrode de contact avec les pieds de l'utilisateur. Dans une version simplifiée, il peut y avoir uniquement deux portions conductrices auquel cas on injecte un courant entre ces deux portions et on mesure une différence de potentiel entre ces deux mêmes portions ; toutefois l'exemple illustré aux figures est basé sur une configuration à quatre portions conductrices 61-64.

La portion conductrice antérieure gauche **61** est destinée à entrer en contact avec l'avant du pied gauche 76 de l'utilisateur ; la portion conductrice antérieure droite **62** est destinée à entrer en contact avec l'avant du pied droit 77; la portion conductrice postérieure droite **63** est destinée à entrer en contact avec le talon du pied droit 77; la portion conductrice postérieure gauche **64** est destinée à entrer en contact avec le talon du pied gauche 76 de l'utilisateur. La configuration inverse est également possible mais moins favorable.

Enfin, le pèse-personne électronique **1** est équipé d'un capteur **7** de concentration de dioxyde de carbone (CO2).

L'afficheur **5** comprend une zone centrale dans laquelle est affiché le poids estimé pour la personne présente sur le pèse-personne **1.** De plus, une zone auxiliaire permet d'afficher des informations auxiliaires, comme la fréquence cardiaque ('FC'), ou la teneur en dioxyde de carbone (CO2) comme il sera décrit ci-après.

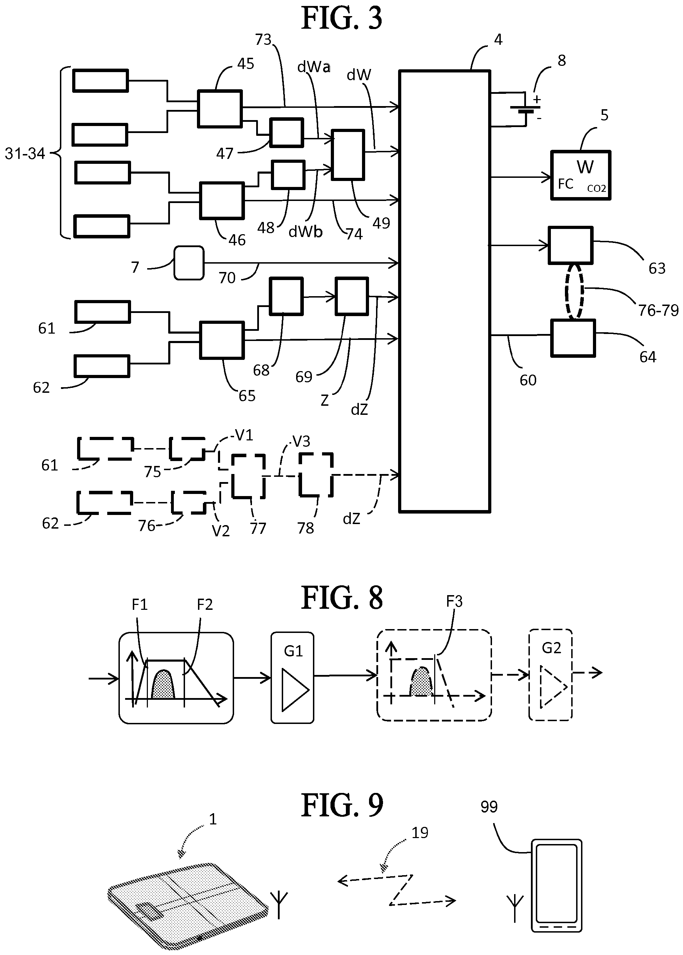

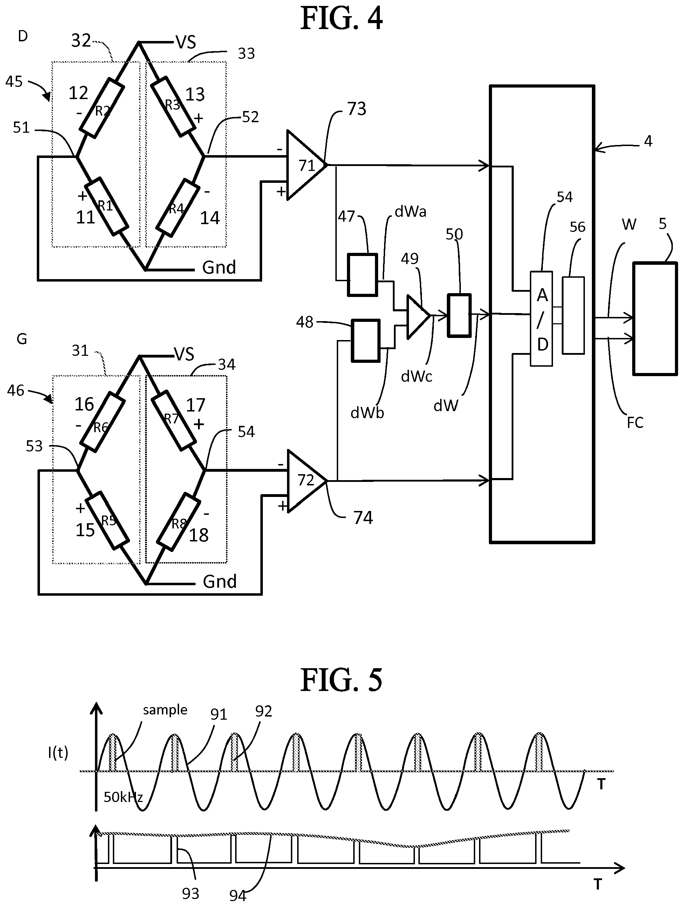

Sur la figure 3, les jauges de contrainte **31-34** sont raccordées à l'unité de commande électronique **4** au moyen de deux montages de type pont de wheatstone **45,46** qui seront détaillés en figure 4. Chacune des sorties **73,74** des ponts de wheatstone est ensuite dirigée vers l'unité de commande électronique **4,** dans laquelle elles sont transformées en mesures numériques par un convertisseur analogique digital **54,** puis sommées par un circuit sommateur **56.** Le poids total **W** en résultant est affiché sur l'afficheur **5.**

Par ailleurs, les sorties **73,74** des ponts de wheatstone **45,46** entrent ensuite respectivement chacun dans un circuit de filtrage et d'amplification **47,48** dont le schéma de principe est détaillé dans la figure 8. La sortie **dWa** du premier circuit de filtrage **47** et la sortie **dWb** du second circuit de filtrage **48** sont additionnées par un montage sommateur **49** qui fournit en sortie un signal **dWc** qui passe par un autre circuit amplificateur **50,** qui à son tour fournit le signal noté **dW,** qui représente un premier signal représentatif des variations de poids périodiques engendrées par les battements cardiaques de l'utilisateur.

En outre, l'unité électronique est alimentée par une batterie **8** de type classique.

De plus, l'unité de commande est raccordée à une des portions conductrices postérieure **63** pour injecter un courant dans un des pieds de l'utilisateur, courant qui passe par les jambes 78, 79 et le corps de l'utilisateur et revient par l'autre pied vers l'autre portion conductrice postérieure **64** qui est reliée à la référence de potentiel **60** de l'unité électronique.

Les deux autres portions conductrices (antérieures) **61** et **62** en contact électrique respectivement avec les portions avant des pieds de l'utilisateur, aux bornes desquels on vient prélever une différence de potentiel, représentative de l'impédance du corps, au moyen d'un circuit différentiel **65.** Ce circuit différentiel 65 fournit en sortie un signal **Z** représentatif de l'impédance mesurée du corps de l'utilisateur. Un second circuit de filtrage et d'amplification **68** permet d'éliminer la composante continue du signal impédance **Z** de filtrer et d'amplifier les variations du signal d'impédance, ce qui donne le signal noté **dZ** qui représente un deuxième signal représentatif des variations d'impédance périodiques engendrées par les battements cardiaques de l'utilisateur.

L'unité électronique est configurée pour déterminer, à partir du premier signal **dW** et du deuxième signal **dZ,** la fréquence cardiaque de l'utilisateur, notée **'Fc',** et qui peut être affichée sur l'afficheur **5.**

La mesure d'impédance **Z** peut être effectuée en injectant un courant prédéterminé entre les portions conductrices 63,64.

Ce courant peut être fixe, ou de préférence pulsé ou modulé. La figure 3 illustre un traitement analogique pour obtenir dZ en préalable au traitement numérique.

De façon avantageuse, il peut être prévu d'injecter un signal alterné comme ceci est illustré sur la figure 5. Dans ce cas, le courant moyen injecté dans l'individu présent sur le pèse-personne est nul. Dans l'exemple illustré, le profil de courant 91,I(t) est sinusoïdal à la fréquence 50 kHz et le courant pic ne dépasse pas 1 milliampère. Le courant peut être fourni par une source de courant alternée, ou bien on peut imposer une tension crête-à-crête.

Selon un traitement de préférence digital, le prélèvement de la tension aux bornes des portions conductrices **61,62** est effectué pendant un temps relativement court correspondant au maximum de l'alternance positive. Le circuit **65** peut donc prélever par échantillonnage périodiquement des mesures **92** représentatives de l'impédance **Z** de l'individu. Dans l'exemple illustré, on prélève le signal pendant 2 ps, la période du signal de commande étant de 20 ps.

Une extrapolation des points échantillonnés **93** permet d'obtenir une courbe **94** correspondant à l'impédance mesurée en fonction du temps.

Selon une alternative de réalisation représentée en trait pointillés à la figure 3, chacune des tensions prélevées sur les portions conductrices **61,62** est tout d'abord traitée séparément par un circuit passe-bande **75** respectivement **76.** Le circuit **75** comprend aussi une fonction amplification et délivre en sortie un premier signal **V1** représentatif des variations de la tension sur la borne 61. Le second circuit 76 délivre de la même façon un second signal **V2** représentatif des variations de la tension sur la borne **62.** Un circuit différenciateur **77** effectue une soustraction des deux tensions V1,V2, et délivre une sortie **V3** qui est amplifiée par un circuit de filtrage et d'amplification **78** ayant un schéma de principe du type de celui décrit en figure 8. À la sortie du circuit 78 on obtient le deuxième signal **dZ** qui représente les variations d'impédance périodiques, converti ensuite en numérique.

La figure 4 montre en détail le schéma électronique relatif à la mesure du poids et de ses variations. Un premier pont de wheatstone **45** combine les résistances **11** et **12** de la jauge de contrainte antérieure droite **32** et les résistances **13** et **14** de la jauge de contrainte postérieure droite **33.** Un premier comparateur **71** met en forme la différence de potentiel entre les points intermédiaires **51** et **52** du premier pont de wheatstone en délivrant une sortie **73** représentative du poids présent sur la partie droite du pèse-personne.

Un second pont de wheatstone **46** combine les résistances **15** et **16** de la jauge de contrainte antérieure gauche **31** et les résistances **17** et **18** de la jauge de contrainte postérieure gauche **34.** Un second comparateur **72** met en forme la différence de potentiel entre les points intermédiaires **53** et **54** du second pont de wheatstone en délivrant une sortie **74** représentative du poids présent sur la partie gauche du pèse-personne.

Les jauges de contrainte **31-34** pourraient bien sûr être combinées selon une logique avant-arrière au lieu d'une logique droite-gauche. Le fait d'amener plusieurs entrées analogiques dans l'unité de commande permet d'indiquer un éventuel décentrage à l'utilisateur. Il est possible d'indiquer un décentrage avant-arrière ou un décentrage gauche-droite, voire même une indication encore plus précise comme cela est présenté dans la demande de brevet FR1256995 du même demandeur.

Par ailleurs, les signaux de pesée partielle 73,74 sont additionnés par le circuit sommateur 47 qui délivre un signal analogique **W** représentatif du poids total de l'utilisateur. Ce signal W entre dans le circuit de filtrage et d'amplification **48** déjà mentionné précédemment. Il délivre en sortie un signal **dW** représentatif des variations de poids déjà mentionné plus haut.

Sur la figure 8, il est décrit la structure et le fonctionnement d'un circuit de filtrage et d'amplification du type de ceux décrits en référence aux circuits 48 et 68.

Un premier filtrage essentiellement passe-bande permet d'éliminer la composante continue du signal ; on utilise typiquement un montage avec un condensateur en série. La fréquence de coupure basse **F1** peut être comprise entre 0,5Hz et 2Hz par exemple. Ce premier filtre inclut également de façon optionnelle une fréquence de coupure haute **F2,** de préférence supérieure à 500 Hz.

En cascade de ce premier filtre, il est prévu un premier étage d'amplification **G1**, de gain compris entre 50 et 2000. En aval de ce premier étage d'amplification, il peut être prévu optionnellement un second filtre avec une fréquence de coupure haute **F3** qui peut être comprise entre 10Hz et 20Hz. En aval de ce second filtre, il peut être prévu optionnellement un second étage d'amplification **G2,** de gain compris entre 10 et 100.

Un tel circuit de filtrage et d'amplification permet d'éliminer de façon très satisfaisante d'une part la composante continue et d'autre part les bruits et autres parasites de fréquence supérieure aux composantes spectrales normalement attendues pour les signaux de variation de poids et/ou d'impédance provoqués par le battement cardiaque.

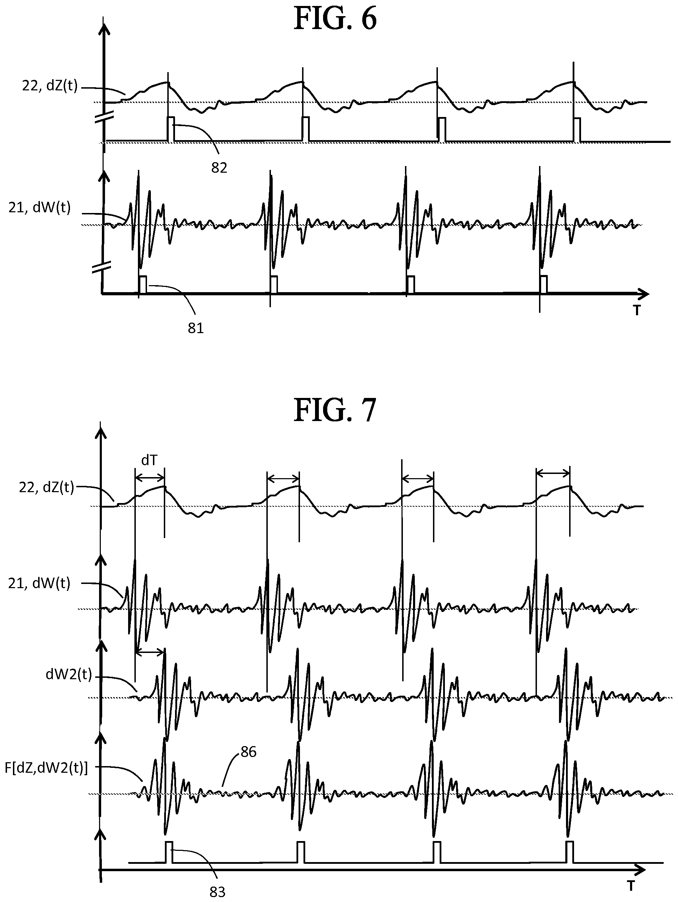

Selon une mise en oeuvre du procédé relativement simple illustrée à la figure 6, le premier signal **21,W(t)** représentatif des variations de poids périodiques engendrées par les battements cardiaques de l'utilisateur est analysé par l'unité de commande, laquelle identifie les maxima pour en déduire un premier signal de pulsation **81.**

L'unité de commande analyse de façon similaire le deuxième signal **22,Z(t),** représentatif des variations d'impédance, pour en extraire les maxima périodiques, afin d'en déduire un second signal de pulsation **82.**

Pour le premier signal, l'unité de commande établit un indice de qualité du signal, par exemple l'amplitude des extrema par rapport à un écart type moyenné du signal, ou encore la médiane des écarts temporels entre les extrema et/ou l'écart-type des écarts temporels entre les extrema ou encore tout autre caractéristique représentant une image du rapport signal sur bruit. L'analyse individuelle du premier signal 21 permet d'obtenir un facteur de qualité noté **FS1.**

L'unité de commande procède de même pour le deuxième signal afin d'établir un indice de qualité du deuxième signal. L'analyse individuelle du second signal 22 permet d'obtenir un facteur de qualité noté **FS2.**

L'unité de commande procède en outre un calcul de corrélation qui sera détaillé plus loin, la sortie de ce calcul de corrélation donnant troisième signal (troisième canal) qui est soumis à une analyse similaire à ce qui décrit précédemment et qui permet d'obtenir un troisième facteur de qualité noté **FS3.**

En fonction de la période constatée et du facteur de qualité calculé respectivement sur chacun des premiers, seconds et troisièmes signaux, l'unité de commande choisit le canal de mesure présumé le plus fiable et affiche la fréquence cardiaque mesurée au moyen de ce canal.

Au lieu de choisir le canal du premier ou deuxième signal à posteriori, on pourrait aussi choisir en temps réel à chaque fois qu'un extrema est détecté, le meilleur signal disponible entre les deux signaux, l'objectif étant de pouvoir délivrer un affichage de la fréquence cardiaque au bout d'un temps d'environ 5 à 10 secondes, de préférence avant 8 secondes.

Comme illustré à la figure 7, s'agissant du calcul de corrélation, on peut procéder tout d'abord à un recalage temporel du premier signal par rapport au second signal ou inversement.

Tout d'abord, l'unité de commande évalue un décalage temporel noté **dT,** au moyen de la position relative des maxima respectivement du premier signal et deuxième signal.

Ensuite, l'un des signaux, en l'occurrence ici le premier signal **21** est retardé d'une valeur temporelle **dT,** afin que les maxima respectifs des signaux soient synchronisés, illustré par le signal **dW2.**

L'unité de commande 4 procède ensuite à un calcul de corrélation, qui consiste en une opération arithmétique réalisée sur les premier et deuxième signaux des signaux, opération notée **F[dZ,dW2].** On peut par exemple choisir de faire une multiplication des signaux entre eux, une addition des signaux ou toute autre opération la plus pertinente au vu de campagnes d'essais sur un échantillon large d'individus d'une population. On peut parler de calcul de corrélation ou d'inter-corrélation.

Dans le signal résultant **86,** les parties bruitées des premier et second signaux ont tendance à s'annuler statistiquement, alors que les portions utiles de signal ont tendance à se renforcer.

À partir de ce signal d'inter-corrélation **86,** l'unité de commande en déduit le troisième signal susmentionné de pulsation **83,** qui dans le cas général présente une meilleure robustesse aux différents aléas et insuffisances de couverture par rapport à l'ensemble des individus de la population.

Il faut noter que l'évaluation d'un indice de qualité tel que décrit plus haut peut également être appliqué à ce troisième signal issu du calcul de corrélation ; on peut ainsi choisir l'un des trois signaux, en particulier si le calcul de corrélation ne s'avère pas de bonne qualité.

Selon une autre variante, en utilisant la puissance de calcul disponible dans l'unité électronique, on peut procéder à des calculs d'inter-corrélation récursifs, en décalant à chaque fois un peu un signal par rapport à l'autre, sans avoir à évaluer **dT** à priori. On choisit alors dans l'ensemble des signaux résultant des calculs d'inter-corrélation, le signal ayant la moyenne la plus forte. Dans la pratique, cela correspondra au recouvrement temporel des parties significatives des signaux. A l'inverse, si les parties significatives des signaux ne se chevauchent pas, alors la multiplication donne un résultat voisin de zéro, car on multiplie un signal significatif par un bruit.

On peut ainsi envisager d'améliorer le temps de réponse pour arriver à moins de 5 secondes, voire moins de 4 secondes.

À titre d'exemple illustratif, il a été conduit des campagnes de mesures statistiques sur plusieurs dizaines de personnes ; un échec est définit comme une erreur de plus de 10% par rapport au rythme cardiaque mesuré par un appareil de référence sur le doigt (par PPG). En utilisant uniquement la mesure des variations de poids on arrive à un taux de 23 % d'échec, en utilisant uniquement la mesure des variations d'impédance on arrive à un taux de 9,6 % d'échec, en utilisant uniquement la corrélation des deux signaux tel qu'explicité ci-dessus, on arrive à 14% d'échec. En sélectionnant la meilleure des 3 techniques (poids/impédance/corrélation) grâce aux facteurs de qualité FS1,FS2,FS3, le taux d'échec global peut être réduit à 3,8%, voire plus petit encore ; un tel résultat ne peut pas être atteint en utilisant un seul des premiers et seconds signaux 21,22.

La figure 9 illustre l'intégration du pèse-personne électronique **1** dans un système de surveillance et suivi de données physiologiques. Dans ce système, le pèse-personne électronique 1 peut transmettre des données moyennant une liaison sans fil **19,** à destination d'un Smartphone **99** (téléphone intelligent) ou d'un ordinateur ; ainsi les données journalières peuvent être accumulées et affichées avec suivi statistique pour un utilisateur ou plusieurs utilisateurs du pèse-personne électronique **1.**

Le procédé mis en oeuvre dans le pèse-personne peut être résumé comme ci-après :
a- on mesure un premier signal 21 représentatif des variations de poids périodiques engendrées par les battements cardiaques de l'utilisateur,
b- on mesure un deuxième signal 22 représentatif des variations d'impédance périodiques engendrées par les battements cardiaques de l'utilisateur,
c- on détermine à partir des premier et deuxième signaux la fréquence cardiaque de l'utilisateur.

L'étape c- peut faire appel à des calculs d'inter-corrélation, soit récursifs, soit simplifiés en ayant tout d'abord procéder à un recalage temporel des deux signaux l'un par rapport à l'autre.

Enfin, l'unité de commande est configurée pour héberger l'électronique de traitement du capteur de concentration de CO2, de corriger les résultats obtenus transmis par ce capteur au moyen de paramètres de calibration ou au moyen des paramètres liés aux circonstances d'utilisation (taux d'humidité, altitude, etc...). La concentration en CO2 peut être affichée, et peut être transmise à des moyens de traitement distant **99** comme évoqué plus haut.

Plus précisément, le capteur de concentration de CO2 est par exemple un capteur de type 'NDIR' (de l'anglais Non Dispersive Infra Red) dans lequel on mesure l'absorption d'un rayonnement infrarouge par la présence des molécules de CO2 dans une cavité parcourue par ledit rayonnement infrarouge. En l'occurrence, l'absorption du CO2 est maximum à la longueur d'onde λ = 4,26 um, pour un premier canal mesurant le taux d'absorption des rayonnements infrarouges. De préférence, on utilise un deuxième canal de mesure dit 'de référence' par exemple à une fréquence différente où l'absorption par le gaz CO2 est négligeable. Avantageusement on compare les mesures du premier canal et du deuxième canal de manière à s'affranchir des dérives, en particulier du vieillissement tant de la source des rayonnements que de la cavité elle-même (encrassement).

Le capteur de concentration de CO2 est relié à l'unité de commande par une liaison multi-filaire **70,** au moyen de laquelle l'unité de commande **4** commande de façon intermittente la source lumineuse et acquiert les signaux des capteurs du premier et du deuxième canal de mesure.

Ainsi, on peut héberger la fonctionnalité de commande du capteur de concentration de CO2 dans l'unité électronique **4** qui réalise déjà les fonctions de pesage et de mesure du rythme cardiaque, de sorte que l'intégration de la fonction de mesure du taux de CO2 est particulièrement poussée.

Par ailleurs, les dimensions du capteur de concentration de CO2 ont été adaptées pour permettre son intégration à l'intérieur d'un pèse-personne électronique. En l'occurrence, le capteur de concentration de CO2 7 se présente comme un tube de diamètre inférieur à 10 mm (voire de préférence inférieur à 8 mm) et de longueur inférieure à 10 cm. Il peut être prévu en outre une communication **71** avec l'air extérieur pour permettre un échange suffisant entre l'air présent dans la cavité et l'air présent dans l'environnement immédiat du pèse-personne.

L'unité de commande 4 est configurée pour déclencher une mesure de concentration du CO2 de façon périodique, par exemple toutes les 10 minutes ou toutes les 30 minutes ou encore selon une périodicité qui peut dépendre de l'heure de la journée ou de la nuit.

Beaucoup de pèse-personnes électroniques sont utilisés dans la chambre à coucher d'une habitation ; dans ce cas, le pèse-personne électronique demeure généralement dans cette pièce ; ainsi il est utilisé grâce à son capteur de concentration de CO2 pour surveiller pendant la nuit l'évolution de la concentration de CO2. En outre, il peut être prévu de transmettre ces données depuis le pèse-personne électronique vers un ordinateur distant ou Smartphone à des fins statistiques de manière analogue à ce qui est prévu pour la fréquence cardiaque.

On peut imaginer un calcul de corrélation entre la fréquence cardiaque mesurée sur un utilisateur lors de son lever avec l'historique de la concentration de CO2 pendant la nuit.

Outre la détermination du rythme cardiaque selon le procédé décrit ci-dessus, on peut aussi utiliser les signaux mesurés pour déterminer la variabilité du rythme cardiaque. Pour ce faire, on utilise plusieurs périodes de signal ; le rythme cardiaque est déterminé comme la moyenne des périodes constatées, tandis que la variabilité du rythme cardiaque est déterminée par un écart type par rapport à cette moyenne.

## Revendications

1. **Dispositif de pesage** de type pèse-personne électronique (1) comprenant au moins **une jauge de contrainte** (31-34), au moins deux portions conductrices (61-64) agencées sur une surface supérieure 6, et une **unité électronique de commande** (4),
la jauge de contrainte étant raccordée à l'unité électronique et l'unité électronique étant configurée pour déterminer le **poids** d'un utilisateur placé sur le dispositif de pesage, et pour mesurer un **premier signal** (21) représentatif des **variations de poids périodiques** engendrées par les battements cardiaques de l'utilisateur,
les portions conductrices étant raccordées à l'unité électronique et l'unité électronique étant configurée pour mesurer une **impédance** aux bornes des pieds de l'utilisateur, et pour mesurer un **deuxième signal** (22) représentatif des variations d'impédance périodiques engendrées par les battements cardiaques de l'utilisateur,
**caractérisé en ce que** l'unité électronique est configurée pour procéder à une **analyse individuelle** qualitative des premier et deuxième signaux et une analyse comparée des premier et deuxième signaux de manière à déterminer à partir des premier et deuxième signaux la fréquence cardiaque de l'utilisateur, à savoir établir pour chaque signal un indice de qualité représentatif du rapport signal sur bruit,
**et en ce que** l'unité électronique est configurée pour réaliser un **calcul** de corrélation sur les premier et deuxième signaux
**et en ce que** l'unité électronique est configurée pour évaluer un indice de qualité du calcul de corrélation, et déterminer la fréquence cardiaque à partir du premier signal et du deuxième signal et du calcul de corrélation, en choisissant celui de ces trois ayant le meilleur indice de qualité.

2. Dispositif de pesage selon la revendication 1, comprenant **quatre portions conductrices** (61-64), dans lequel l'unité électronique est configurée pour injecter un courant pulsé ou modulé entre deux des portions conductrices (62,63) et mesurer la différence de potentiel entre les deux autres portions conductrices (61-64), pour déterminer les variations de différence de potentiel, et déterminer ainsi le deuxième signal.

3. Dispositif de pesage selon la revendication 2, dans lequel l'unité électronique est configurée pour mesurer un **déphasage temporel** (dT) entre les premier et deuxième signaux, et décaler le deuxième signal par rapport au premier signal dudit déphasage temporel, de manière à ce que le calcul de corrélation élimine les bruits et maximise les portions de signal utiles.

4. Dispositif de pesage selon l'une des revendications 1 à 3, comprenant **quatre pieds** (41-44) et **quatre jauges** de contrainte (31-34) correspondantes, combinées dans deux ponts de wheatstone, pour permettre à l'unité électronique de mesurer le poids d'un utilisateur et ses variations.

5. **Procédé** mis en œuvre dans un pèse-personne électronique comprenant au moins une jauge de contrainte, au moins deux portions conductrices (61-64) agencées sur une surface supérieure (6), et une unité électronique de commande (4),
la jauge de contrainte et les portions conductrices (61-64) étant raccordées à l'unité électronique, pour permettre à l'unité électronique de mesurer le poids d'un utilisateur placé sur le dispositif de pesage, et une impédance aux bornes des pieds de l'utilisateur,
le procédé comprenant les étapes :
a- mesurer un premier signal (21) représentatif des variations de poids périodiques engendrées par les battements cardiaques de l'utilisateur,
b- mesurer un deuxième signal (22) représentatif des variations d'impédance périodiques engendrées par les battements cardiaques de l'utilisateur,
c0- procéder à une analyse individuelle qualitative des premier et deuxième signaux et une analyse comparée des premiers et deuxième signaux, à savoir établir pour chaque signal un indice de qualité représentatif du rapport signal sur bruit,
c- réaliser au moins un calcul de corrélation sur les premier et deuxième signaux,
déterminer à partir des premier et deuxième signaux et du calcul de corrélation la fréquence cardiaque de l'utilisateur, en choisissant celui de ces trois ayant le meilleur indice de qualité.

6. Procédé selon la revendication 5, dans lequel le dispositif comprend quatre portions conductrices (61-64), le procédé comprenant au cours de l'étape b-:
injecter un courant pulsé entre deux des portions conductrices (62,63) et mesurer la différence de potentiel entre les deux autres portions conductrices (61-64), pour déterminer les variations de différence de potentiel, et déterminer ainsi le deuxième signal.

7. Procédé selon la revendication 6, comprenant en outre dans l'étape c- :
mesurer un déphasage temporel entre les premier et deuxième signaux, et décaler le deuxième signal par rapport au premier signal dudit déphasage temporel, de manière à ce que le calcul de corrélation élimine les bruits et maximise les portions de signal utiles.

## Patentansprüche

1. Wiegevorrichtung des Typs elektronische Personenwaage (1), aufweisend mindestens einen Dehnungsmesser (31-34), mindestens zwei auf einer oberen Oberfläche 6 angeordnete leitfähige Abschnitte (61-64) und eine elektronische Steuereinheit (4), wobei
der Dehnungsmesser mit der elektronischen Einheit verbunden ist und die elektronische Einheit konfiguriert ist, das Gewicht eines auf der Wiegevorrichtung angeordneten Benutzers zu bestimmen und ein erstes Signal (21) zu messen, das von den Herzschlägen des Benutzers hervorgerufene periodische Gewichtsvariationen repräsentiert,
die leitfähigen Abschnitte mit der elektronischen Einheit verbunden sind und die elektronische Einheit konfiguriert ist, eine Impedanz an den Anschlüssen der Füße des Benutzers zu messen und ein zweites Signal (22) zu messen, das von den Herzschlägen des Benutzers hervorgerufene periodische Impedanzvariationen repräsentiert,
**dadurch gekennzeichnet, dass** zur Bestimmung der Herzfrequenz des Benutzers ausgehend von dem ersten und zweiten Signal die elektronische Einheit konfiguriert ist, eine qualitative Einzelanalyse des ersten und zweiten Signals und eine das erste und zweite Signal vergleichende Analyse vorzunehmen, um für jedes Signal einen Qualitätsindex, der das Signal/Rausch-Verhältnis repräsentiert, erstellen zu können,
und dadurch, dass die elektronische Einheit konfiguriert ist, eine Korrelationsberechnung an dem ersten und zweiten Signal durchzuführen,
und dadurch, dass die elektronische Einheit konfiguriert ist, einen Qualitätsindex der Korrelationsberechnung zu evaluieren und ausgehend von dem ersten und dem zweiten Signal und der Korrelationsberechnung die Herzfrequenz dadurch zu bestimmen, dass aus diesen drei Größen diejenige mit dem besten Qualitätsindex ausgewählt wird.

2. Wiegevorrichtung nach Anspruch 1, aufweisend vier leitfähige Abschnitte (61-64), in welcher die elektronische Einheit konfiguriert ist, einen gepulsten oder modulierten Strom zwischen zwei der leitfähigen Abschnitte (62, 63) einzuspeisen und die Potentialdifferenz zwischen den zwei anderen leitfähigen Abschnitten (61-64) zu messen, um die Potentialdifferenzvariationen zu bestimmen und auf diese Weise das zweite Signal zu bestimmen.

3. Wiegevorrichtung nach Anspruch 2, in welcher die elektronische Einheit konfiguriert ist, einen zeitlichen Phasenversatz (dT) zwischen dem ersten und zweiten Signal zu messen und das zweite Signal bezüglich des ersten Signals um den zeitlichen Phasenversatz zu verschieben, so dass die Korrelationsberechnung das Rauschen eliminiert und die Nutzsignalanteile maximiert.

4. Wiegevorrichtung nach einem der Ansprüche 1 bis 3, aufweisend vier Füße (41-44) und vier entsprechende Dehnungsmesser (31-34), die in zwei Wheatstone-Brückenschaltungen kombiniert sind, um der elektronischen Einheit zu erlauben, das Gewicht eines Benutzers und dessen Variationen zu messen.

5. Verfahren, das in einer elektronischen Personenwaage durchgeführt wird, die mindestens einen Dehnungsmesser, mindestens zwei auf einer oberen Oberfläche (6) angeordnete leitfähige Abschnitte (61-64) und eine elektronische Steuereinheit (4), aufweist,
wobei der Dehnungsmesser und die leitfähigen Abschnitte (61-64) mit der elektronischen Einheit verbunden sind, um der elektronischen Einheit zu erlauben, das Gewicht eines auf der Wiegevorrichtung angeordneten Benutzers und eine Impedanz an den Anschlüssen der Füße des Benutzers zu messen,
wobei das Verfahren die Schritte aufweist:
a- Messen eines ersten Signals (21), das von den Herzschlägen des Benutzers hervorgerufene periodische Gewichtsvariationen repräsentiert,
b- Messen eines zweiten Signals (22), das von den Herzschlägen des Benutzers hervorgerufene periodische Impedanzvariationen repräsentiert,
c0- Vornehmen einer qualitativen Einzelanalyse des ersten und zweiten Signals und einer das erste und zweite Signal vergleichenden Analyse, um für jedes Signal einen Qualitätsindex, der das Signal/Rausch-Verhältnis repräsentiert, erstellen zu können,
c- Durchführen mindestens einer Korrelationsberechnung an dem ersten und zweiten Signal,
Bestimmen der Herzfrequenz des Benutzers ausgehend von dem ersten und zweiten Signal und der Korrelationsberechnung durch Auswählen derjenigen der drei Größen, die den besten Qualitätsindex hat.

6. Verfahren nach Anspruch 5, in welchem die Vorrichtung vier leitfähige Abschnitte (61-64) aufweist, und das Verfahren im Verlauf des Schritts b- aufweist:
Einspeisen eines gepulsten Stroms zwischen zwei der leitfähigen Abschnitte (62, 63) und Messen der Potentialdifferenz zwischen den zwei anderen leitfähigen Abschnitten (61-64), um die Potentialdifferenzvariationen zu bestimmen und auf diese Weise das zweite Signal zu bestimmen.

7. Verfahren nach Anspruch 6, aufweisend ferner in dem Schritt c-:
Messen eines zeitlichen Phasenversatzes zwischen dem ersten und zweiten Signal und Verschieben des zweiten Signals bezüglich des ersten Signals um den zeitlichen Phasenversatz, so dass die Korrelationsberechnung das Rauschen eliminiert und die Nutzsignalanteile maximiert.

## Claims

1. Weighing device (1) of the digital scale type, comprising at least one **strain gauge (31-34),** at least **two conducting portions (61-64)** arranged on a top surface (6), and an electronic control unit (4),
the strain gauge being connected to the electronic unit and the electronic unit being configured to determine the weight of a user positioned on the weighing device, and to measure a **first signal (21)** indicative of the periodic **weight variations** caused by the heartbeats of the user,
the conducting portions being connected to the electronic unit and the electronic unit being configured to measure an **impedance** at the user's feet and to measure a **second signal (22)** indicative of the periodic **impedance variations** caused by the heartbeats of the user,
**characterized in that** the electronic unit is configured to perform a qualitative individual analysis of the first and second signals and a comparative analysis of the first and second signals in order to determine from the first and second signals the heart rate of the user, that is to establish for each signal a quality index representative of the signal to noise ratio, and **in that** the electronic unit is configured to perform a correlation calculation on the first and second signals,
and **in that** the electronic unit is configured to evaluate a quality index for the correlation calculation, and determine the heart rate from the first signal and the second signal and the correlation calculation by choosing the one of the three having the best quality index.

2. Weighing device according to claim 1 comprising **four** conducting portions (61-64), wherein the electronic unit is configured to inject a modulated or pulsed current between two of the conducting portions (62, 63) and to measure the electric potential difference between the two other conducting portions (61-64), in order to determine the variations in electric potential difference and thus determine the second signal.

3. Weighing device according to claim 2, wherein the electronic unit is configured to measure a phase difference (dT) between the first and second signals, and to shift the second signal relative to the first signal by said phase difference so that the cross-correlation calculation eliminates noise and maximizes the useful signal portions.

4. Weighing device according to any of claims 1 to 3, comprising four feet (41-44) and four corresponding strain gauges (31-34), combined into two Wheatstone bridges, to allow the electronic unit to measure a user's weight and weight variations.

5. **Method** implemented in a digital scale comprising at least one **strain gauge,** at least two **conducting portions** (61-64) arranged on a top surface (6), and an electronic control unit (4),
the strain gauge and the conducting portions (61-64) being connected to the electronic unit to enable the electronic unit to measure the weight of a user positioned on the weighing device and an impedance at the terminals of the **user's feet,**
the method comprising the steps of:
a- measure a first signal (21) indicative of the periodic variations in weight caused by the heartbeats of the user,
b- measure a second signal (22) indicative of the periodic variations in impedance caused by the heartbeats of the user,
c0- performing a qualitative individual analysis of the first and second signals and a comparative analysis of the first and second signals, that is to establish for each signal a quality index representative of the signal to noise ratio,
c - perform at least one correlation calculation on the first and second signal,
- determine from the first and second signals and from the correlation calculation the heart rate of the user, by choosing the one of the three having the best quality index.

6. Method according to claim 5, wherein the device comprises four conducting portions (61-64), said method comprising during step b:
injecting a pulsed current between two of the conducting portions (62, 63) and measuring the electric potential difference between the two other conducting portions (61-64), in order to determine the variations in electric potential difference and thus determine the second signal.

7. Method according to claim 6, further comprising in step c:
measuring a phase difference between the first and second signals, and shifting the second signal relative to the first signal by said phase difference, so that the cross-correlation calculation eliminates noise and maximizes the useful signal portions.
